# EUROPÄISCHE PATENTSCHRIFT

(11) **EP 1 723 101 B1**
(45) Veröffentlichungstag und Bekanntmachung des Hinweises auf die Patenterteilung: **09.09.2009**
(21) Anmeldenummer: 05701505.9
(22) Anmeldetag: 13.01.2005
(51) Int. Cl.: C07C 267/00

(54) **VERFAHREN ZUR HERSTELLUNG VON CARBODIIMIDGRUPPEN AUFWEISENDEN SUBSTANZEN**
METHOD FOR THE PRODUCTION OF SUBSTANCES COMPRISING CARBODIIMIDE GROUPS
PROCÉDÉ POUR PRODUIRE DES SUBSTANCES COMPORTANT DES GROUPES CARBODIIMIDES

(30) Priorität: 09.03.2004 DE 102004011791
(43) Veröffentlichungstag der Anmeldung: 22.11.2006
(73) Patentinhaber: Evonik Degussa GmbH, 45128 Essen (DE)
(72) Erfinder: SPYROU, Emmanouil, 46514 Schermbeck (DE); EIKEMPER, Bernhard, 46240 Bottrop (DE)
(86) Internationale Anmeldenummer: PCT/EP2005/050119
(87) Internationale Veröffentlichungsnummer: WO 2005/087716

(56) Entgegenhaltungen:
- EP-A- 0 628 541
- US-A- 2 840 589

## Beschreibung

Die Erfindung betrifft ein Verfahren zur Herstellung von Carbodiimidgruppen tragenden Substanzen.

Carbodiimidgruppen tragende Substanzen sind als Vernetzer für Carbonsäuregruppen tragende Polymere gut geeignet Sie werden daher unter anderem für die Vernetzung von carboxylhaltigen Latices eingesetzt (US 4 419 294, US 482 063). Außerdem finden sie Einsatz als Feuchtigkeitsfänger in Reaktivsystemen.

Die Herstellung von Carbodiimidgruppen aus Isocyanaten ist bekannt und beispielsweise in US 2 840 589 und US 2 941 966 beschrieben worden. Allen gängigen Verfahren heutzutage ist gemein, dass als Katalysatoren Verbindungen des Phosphors verwendet werden, wie z. B. 1-Methyl-phospholen-1-oxid. Solche Phosphorverbindungen sind toxisch und häufig auch kanzerogen. Die Herstellung sowie der Einsatz solcher toxischen Phosphorverbindungen stellt ein arbeitshygienisches Problem dar.

Aufgabe der Erfindung war es daher, aus Isocyanaten carbodiimidgruppenhaltige Substanzen herzustellen, ohne auf giftige oder kanzerogene Katalysatoren zurückgreifen zu müssen.

Überraschend konnte diese Aufgabe gelöst werden durch ein Verfahren unter Verwendung von Wasser und/oder Wasser enthaltenden oder abgebenden Stoffen, und/oder Aminen und/oder Harnstoffen, als Katalysatoren.

Gegenstand der vorliegenden Erfindung ist ein Verfahren zur Herstellung von Carbodiimid tragenden Substanzen aus Isocyanaten unter Verwendung von Wasser und/oder Wasser enthaltenden oder abgebenden Stoffen, und/oder Aminen und/oder Harnstoffen, als Katalysatoren.

Bevorzugter Gegenstand der vorliegenden Erfindung ist ein Verfahren zur Herstellung von Carbodiimid tragenden Substanzen aus Isocyanaten erhalten durch Umsetzung einer Mischung aus
A) mindestens einer Ausgangsverbindung mit mindestens einer Isocyanatgruppe,
B) mindestens einem Katalysator in einer Menge von 0,01 bis 30 Gew.-%, bezogen auf die Summe aus A) und B), ausgewählt aus
   1. Wasser,
   2. Wasser enthaltenden und/oder Wasser abgebenden Stoffen,
   3. primären und/oder sekundären Aminen,
   4. Harnstoffen mit der Struktur R¹-NH-CO-NR³R³, wobei R¹, R² und R³ gleiche oder verschiedene Reste mit 1 bis 15 Kohlenstoffatome oder H bedeuten,
C) gegebenenfalls einen oder mehrere Co-Katalysatoren aus der Gruppe der metallhaltigen Substanzen in einer Menge von 0,00001 bis 1 Gew.-%, bezogen auf die Summe von A) und B),
wobei die Mischung aus A), B) und gegebenenfalls C) 5 Minuten bis 12 Stunden bei einer Temperatur von 120 bis 300 °C und bei Drücken zwischen 1 und 25 bar gehalten wird.

Erfindungsgemäß können als Komponente A) alle Isocyanat tragenden Substanzen eingesetzt werden, wie z. B. Cyclohexylisocyanat, Isophorondiisocyanat (IPDI), Hexamethylendiisocyanat (HDI), 2-Methylpentandiisocyanat (MPDI), 2,2,4-Trimethylhexamethylendiisocyanat/2,4,4-Trimethyl-hexamethylendiisocyanat (TMDI), Norbornandiisocyanat (NBDI), Methylendiphenyldiisocyanat (MDI), Diisocyanatomethylbenzen, insbesondere das 2,4- und das 2,6-Isomere und technische Gemische beider Isomeren (TDI), Tetramethylxylylendiisocyanat (TMXDI) und Dicyclohexylmehtyldiisocyanat (H12MDI). Bevorzugt werden IPDI, HDI und H12MDI verwendet. Auch Derivate der vorstehenden Isocyanate, wie z. B. Isocyanurate, Uretdione, Allophanate und/oder Biurete sind geeignet

Als Katalysatoren B) kommen alle Substanzen in Frage, die entweder Wasser enthalten oder während der Umsetzung Wasser freisetzen.

In Frage kommen als Katalysatoren B1) Wasser sowie B2) Wasser enthaltende und Wasser abgebende Stoffe, wie z. B. anorganische Verbindungen mit Kristallwasser, Molekularsiebe, Ionenaustauscher, wasserhaltige Polymergele, wie z. B. Superabsorber.
Als Katalysatoren B3) sind primäre oder sekundäre Amine, wie z. B. Cyclohexylamin, Methylamin, Ethylamin, Butylamin, Dimethylamin, Diethylamin, Dibutylamin geeignet Besonders bevorzugt werden sekundäre Amine, wie z.B. Cyclohexylamin.

Als Harnstoffe B4) sind alle Verbindungen mit der Struktur R¹-NH-CO-NR²R³, einsetzbar, wobei R¹, R² und R³ gleiche oder verschiedene Reste mit 1 bis 15 Kohlenstoffatomen oder H bedeuten. Insbesondere können die Reste R¹ bis R³ gleichzeitig oder unabhängig voneinander Methyl, Ethyl, Propyl, Butyl, Pentyl, Hexyl, Cyclohexyl bedeuten, wobei die homologe Reihe bis zu Alkylresten mit 15 Kohenstoffatomen fortzuführen ist Es können auch Harnstoffe mit aromatischen Resten eingesetzt werden. Besonders bevorzugt ist Dicyclohexylharnstoff.

Als Wasser enthaltende Stoffe sind zum Beispiel Molekularsiebe besonders gut geeignet Molekularsiebe sind chemisch gesehen Zeolithe. Zeolithe ist laut Römpps Chemie Lexikon (Thieme Verlag, Stuttgart, 1999) eine "abgeleitete Bezeichnung für eine weit verbreitete Gruppe von kristallinen Silicaten, und zwar von wasserhaltigen Alkali- bzw. Erdalkali-Alumosilicaten (ähnlich den Feldspäten) der allgemeinen Formel M₂/_{z}O × Al₂O₃ × xSiO₂ × yH₂O, wobei M = ein- oder mehrwertiges Metall (meist ein Alkali- oder Erdalkali-Kation), H oder NH₄ u. a., z = Wertigkeit des Kations, x = 1,8 bis ca. 12 und y = 0 bis ca. 8. Das stöchiometrische Verhältnis von SiO₂ zu Al₂O₃ (Modul) ist eine wichtige Kenngröße der Zeolithe. Charakteristisch für die meisten Zeolithe ist, dass sie ihr Wasser beim Erhitzen stetig und ohne Änderung der Kristallstruktur abgeben und andere Verbindungen anstelle des entfernten Wassers aufnehmen." Zeolithe können im Chemikalienhandel (z. B. Aldrich) unter der Bezeichnung Molekularsiebe bezogen werden.

Als Co-Katalysatoren C) eignen sich metallhaltigen Substanzen, vor allem solche auf Basis Zinn, Zink und Wismut, wie z. B. Zinn(II)chlorid, Dibutylzinndilaurat, Zinkoctoat, Zinkacetylacetonat und Wismut-neo-dodecanoat. Besonders bevorzugt wird Zinn(II)chlorid. Solche Co-Katalysatoren werden in Mengen von 0,00001 bis 1 % eingesetzt.

Das Verfahren läuft so ab, dass zunächst alle Komponenten A), B) und gegebenenfalls C) bei Raumtemperatur miteinander vermischt werden. Im Falle von festen Isocyanaten wird eine Mischungstemperatur oberhalb des Schmelzpunkts gewählt. Daraufhin wird die Mischung auf eine Temperatur zwischen 120 und 300 °C gebracht und 5 Minuten bis 12 Stunden bei dieser Temperatur gehalten. Bevorzugt werden Temperaturen zwischen 200 und 250 °C und Reaktionszeiten zwischen 1 und 6 Stunden. Gegebenenfalls kann diese Reaktion auch in einem Druckbehälter bei Drücken zwischen 1 und 25 bar durchgeführt werden. Nach vollendeter Reaktion kann gegebenenfalls nicht umgesetzte Komponente A) durch geeignete Trennverfahren abgetrennt werden. In Frage kommen dabei destillative Verfahren, vor allem Kurz- und Dünnschichtdestillationen.

Im Folgenden wird die Erfindung durch experimentelle Beispiele näher erläutert, ohne sie damit einzuschränken.

### Beispiele

Als Einsatzstoffe werden folgende Komponenten verwendet:

| **Einsatzstoffe** | Hersteller |
|---|---|
| Cyclohexylisocyanat | Aldrich |
| Cylohexylamin | Aldrich |
| Dicyclohexylaminharnstoff | Aldrich |
| Molekularsieb (0,5 nm) | Merck, Perlform 2 mm |
| Zinn(II)chlorid | Aldrich |
| Dicyclohexylcarbodiimid | Aldrich |

### Allgemeine Versuchsdurchführung

Die Einsatzstoffe werden bei Raumtemperatur vermischt und dann in einem Autoklaven 4 Stunden bei 230°C gehalten. Danach wird die Reaktionsmischung auf Raumtemperatur abgekühlt und mit Hilfe der Gaschromatographie untersucht

**Angaben in Gew.-%**

| | 1 | 2 | 3 | 4 | 5 | V1* | V2* |
|---|---|---|---|---|---|---|---|
| Cyclohexylisocyanat | 99,9 | 99,7 | 99,5 | 95,0 | 99,9 | 100 | 100 |
| Wasser | 0,1 | | | | 0,1 | | |
| Cyclohexylamin | | 0,3 | | | | | |
| Dicyclohexylaminharnstoff | | | 0,5 | | | | |
| Molekularsieb (0,5 nm) | | | | 5,0 | | | |
| Zinn(II)chlorid | | | | | 0,004 | | 0,004 |
| Analyse des Reaktionsprodukts: Dicyclohexylcarbodiimid (GC, Flächen-%) | 15,9 | 9,6 | 8,9 | 30,8 | 21,5 | 3,4 | 4,3 |

| | | | | | | | |
|---|---|---|---|---|---|---|---|
| * Dies sind nicht erfindungsgemäße Vergleichsbeispiele. | | | | | | | |

## Patentansprüche

1. Verfahren zur Herstellung von Carbodiimid tragenden Substanzen aus Isocyanaten unter Verwendung von Wasser und/oder Wasser enthaltenden oder abgebenden Stoffen, und/oder Aminen und/oder Harnstoffen, als Katalysatoren.

2. Verfahren zur Herstellung von Carbodiimid tragenden Substanzen aus Isocyanaten erhalten durch Umsetzung einer Mischung aus
A) mindestens einer Ausgangsverbindung mit mindestens einer Isocyanatgruppe,
B) mindestens einem Katalysator in einer Menge von 0,01 bis 30 Gew.%, bezogen auf die Summe aus A) und B), ausgewählt aus
1. Wasser,
2. Wasser enthaltenden und/oder Wasser abgebenden Stoffen,
3. primären und/oder sekundären Aminen,
4. Harnstoffen mit der Struktur R¹-NH-CO-NR³R³, wobei R¹, R² und R³ gleiche oder verschiedene Reste mit 1 bis 15 Kohlenstoffatome oder H bedeuten,
C) gegebenenfalls einen oder mehrere Co-Katalysatoren aus der Gruppe der metallhaltigen Substanzen in einer Menge von 0,00001 bis 1 Gew.%, bezogen auf die Summe von A) und B),
wobei die Mischung aus A), B) und gegebenenfalls C) 5 Minuten bis 12 Stunden bei einer Temperatur von 120 bis 300 °C und bei Drücken zwischen 1 und 25 bar gehalten wird.

3. Verfahren nach mindestens einem der vorherigen Ansprüche,
**dadurch gekennzeichnet,**
**dass** als Komponente A) Cyclohexylisocyanat, Isophorondiisocyanat (IPDI), Hexamethylendiisocyanat (HDI), 2-Methylpentandiisocyanat (MPDI), 2,2,4-Trimethylhexamethylendiisocyanat/2,4,4- Trimethyl-hexamethylendiisocyanat (TMDI), Norbornandiisocyanat (NBDI), Methylendiphenyldiisocyanat (MDI), Diisocyanatomethylbenzen, insbesondere das 2,4- und das 2,6-Isomere und technische Gemische beider Isomeren (TDI), Tetramethylxylylendiisocyanat (TMXDI) und Dicyclohexylmehtyldiisocyanat (H12MDI) eingesetzt werden.

4. Verfahren nach Anspruch 3,
**dadurch gekennzeichnet,**
**dass** IPDI, HDI und/oder H12MDI eingesetzt werden.

5. Verfahren nach mindestens einem der vorherigen Ansprüche,
**dadurch gekennzeichnet,**
**dass** Isocyanurate, Uretdione, Allophanate und/oder Biurete als Isocyanate eingesetzt werden.

6. Verfahren nach mindestens einem der vorherigen Ansprüche,
**dadurch gekennzeichnet,**
**dass** als Komponente B2) anorganische Verbindungen mit Kristallwasser, Molekularsiebe, Ionenaustauscher, wasserhaltige Polymergele, wie z. B. Superabsorber, eingesetzt werden.

7. Verfahren nach mindestens einem der vorherigen Ansprüche,
**dadurch gekennzeichnet,**
**dass** als Amine B3) Cyclohexylamin, Methylamin, Ethylamin, Butylamin, Dimethylamin, Diethylamin, Dibutylamin eingesetzt werden.

8. Verfahren nach mindestens einem der vorherigen Ansprüche,
**dadurch gekennzeichnet,**
**dass** als Harnstoff B4) Dicyclohexylharnstoff eingesetzt wird.

9. Verfahren nach mindestens einem der vorherigen Ansprüche,
**dadurch gekennzeichnet,**
**dass** Co-Katalysatoren C) auf Basis von Zinn, Zink und/oder Wismut eingesetzt werden.

10. Verfahren nach Anspruch 9,
**dadurch gekennzeichnet,**
**dass** Zinn(II)chlorid, Dibutylzinndilaurat, Zinkoctoat, Zinkacetylacetonat und Wismut-neo-dodecanoat allein oder in Mischungen eingesetzt werden.

## Claims

1. Process for preparing substances bearing carbodiimide from isocyanates using water and/or water-containing or -releasing substances, and/or amines and/or ureas as catalysts.

2. Process for preparing substances bearing carbodiimide from isocyanates obtained by reacting a mixture of
A) at least one starting compound having at least one isocyanate group,
B) at least one catalyst in an amount of from 0.01 to 30% by weight, based on the sum of A) and B), selected from
1. water,
2. water-containing and/or water-releasing substances,
3. primary and/or secondary amines,
4. ureas having the structure R¹-NH-CO-NR³R³, where R¹, R² and R³ are each identical or different radicals having from 1 to 15 carbon atoms or H,
C) optionally one or more cocatalysts from the group of metal-containing substances in an amount of from 0.00001 to 1% by weight, based on the sum of A) and B),
by holding the mixture of A), B) and, where present, C) at a temperature of from 120 to 300°C and at pressures between 1 and 25 bar for from 5 minutes to 12 hours.

3. Process according to at least one of the preceding claims,
**characterized in that**
the component A) used is cyclohexyl isocyanate, isophorone diisocyanate (IPDI), hexamethylene diisocyanate (HDI), 2-methylpentane diisocyanate (MPDI), 2,2,4-trimethylhexamethylene diisocyanate/2,4,4-trimethylhexamethylene diisocyanate (TMDI), norbornane diisocyanate (NBDI), methylenediphenyl diisocyanate (MDI), diisocyanatomethylbenzene, especially the 2,4- and the 2,6-isomers, and technical grade mixtures of both isomers (TDI), tetramethylxylylene diisocyanate (TMXDI) and dicyclohexylmethyl diisocyanate (H12MDI).

4. Process according to Claim 3,
**characterized in that**
IPDI, HDI and/or H12MDI are used.

5. Process according to at least one of the preceding claims,
**characterized in that**
the isocyanates used are isocyanurates, uretdiones, allophanates and/or biurets.

6. Process according to at least one of the preceding claims,
**characterized in that**
the component B2) used comprises inorganic compounds having water of crystallization, molecular sieves, ion exchangers, hydrous polymer gels, for example superabsorbents.

7. Process according to at least one of the preceding claims,
**characterized in that**
the amines B3) used are cyclohexylamine, methylamine, ethylamine, butylamine, dimethylamine, diethylamine, dibutylamine.

8. Process according to at least one of the preceding claims,
**characterized in that**
the urea B4) used is dicyclohexylurea.

9. Process according to at least one of the preceding claims,
**characterized in that**
cocatalysts C) based on tin, zinc and/or bismuth are used.

10. Process according to Claim 9,
**characterized in that**
tin(II) chloride, dibutyltin dilaurate, zinc octoate, zinc acetylacetonate and bismuth neododecanoate are used alone or in mixtures.

## Revendications

1. Procédé pour la préparation de substances portant du carbodiimide à partir d'isocyanates avec utilisation d'eau et/ou de substances contenant ou libérant de l'eau, et/ou des amines et/ou des urées comme catalyseurs.

2. Procédé pour la préparation de substances portant du carbodiimide à partir d'isocyanates, obtenues par transformation d'un mélange constitué par
A) au moins un composé de départ comprenant au moins un groupe isocyanate,
B) au moins un catalyseur en une quantité de 0,01 à 30% en poids, par rapport à la somme de A) et B), choisi parmi
1. l'eau,
2. des substances contenant et/ou libérant de l'eau,
3. des amines primaires et/ou secondaires,
4. des urées présentant la structure R¹-NH-CO-NR³R³, où R¹, R² et R³ signifient des radicaux identiques ou différents comprenant 1 à 15 atomes de carbone ou H,
C) le cas échéant un ou plusieurs cocatalyseurs du groupe des substances contenant du métal en une quantité de 0,00001 à 1% en poids, par rapport à la somme de A) et B),
où le mélange de A), B) et le cas échéant C) est maintenu pendant 5 minutes à 12 heures à une température de 120 à 300°C et à des pressions entre 1 et 25 bars.

3. Procédé selon au moins l'une quelconque des revendications précédentes, **caractérisé en ce qu'**on utilise comme composant A) du cyclohexylisocyanate, de l'isophoronediisocyanate (IPDI), de l'hexaméthylènediisocyanate (HDI), du 2-méthylpentanediisocyanate (MPDI), du 2,2,4-triméthylhexaméthylènediisocyanate/2,4,4-triméthylhexaméthylènediisocyanate (TMDI), du norbornanediisocyanate (NBDI), du méthylènediphényldiisocyanate (MDI), du diisocyanatométhylbenzène, en particulier l'isomère 2,4 et l'isomère 2,6 et les mélanges de qualité technique des deux isomères (TDI), du tétraméthylxylylènediisocyanate (TMXDI) et du dicyclohexylméthyldiisocyanate (H12MDI).

4. Procédé selon la revendication 3, **caractérisé en ce qu'**on utilise de l'IPDI, de l'HDI et/ou du H12MDI.

5. Procédé selon au moins l'une quelconque des revendications précédentes, **caractérisé en ce qu'**on utilise des isocyanurates, des uretdiones, des allophanates et/ou des biurets comme isocyanates.

6. Procédé selon au moins l'une quelconque des revendications précédentes, **caractérisé en ce qu'**on utilise comme composant B2) des composés inorganiques avec de l'eau de cristallisation, des tamis moléculaires, des échangeurs d'ions, des gels polymères contenant de l'eau, tels que par exemple des superabsorbants.

7. Procédé selon au moins l'une quelconque des revendications précédentes, **caractérisé en ce qu'**on utilise comme amines B3) de la cyclohexylamine, de la méthylamine, de l'éthylamine, de la butylamine, de la diméthylamine, de la diéthylamine, de la dibutylamine.

8. Procédé selon au moins l'une quelconque des revendications précédentes, **caractérisé en ce qu'**on utilise comme urée B4) de la dicyclohexylurée.

9. Procédé selon au moins l'une quelconque des revendications précédentes, **caractérisé en ce qu'**on utilise des cocatalyseurs C) à base d'étain, de zinc et/ou de bismuth.

10. Procédé selon la revendication 9, **caractérisé en ce qu'**on utilise du chlorure d'étain (II), du dilaurate de dibutylétain, de l'octoate de zinc, de l'acétylacétonate de zinc et du néododécanoate de bismuth, seuls ou en mélanges.
